# EUROPEAN PATENT APPLICATION

(11) **EP 0 577 179 A1**
(43) Date of publication of application: **05.01.1994**
(21) Application number: 93201763.5
(22) Date of filing: 18.06.1993
(51) Int. Cl.: A61F 2/44

(54) **An implant for fixing adjacent vertebra**

(30) Priority: 20.06.1992 DE 4220215
(71) Applicant: ESKA Implants GmbH & Co., 23556 Lübeck (DE)
(72) Inventor: Schug, Martin, D-23552 Lübeck (DE); Rischke, Burkhard, D-25462 Rellingen (DE)
(74) Representative: Weiss, Christian, Dipl.-Ing.

(57) **Abstract**

An implant (1) for fixing adjacent vertebrae in the region of the opposing bearing surfaces of the vertebrae is provided, in which several spherical spacers (2) having an open-cell or open-pore metal structure are disposed on at least one connecting element (6).

## Description

The invention relates to an implant for fixing adjacent vertebrae in the region of the opposing surfaces of the body of the vertebrae, characterised by several spherical spacers having an open-cell or open-pore metal structure, which is disposed on at least one connecting element.

Such an implant is for example known from EP-A-O 271 501, which consists of a cylindrical block having an open-cell or open-pore surface structure and is implanted in a recess, which is resected at the opposing bone faces of adjacent vertebral bodies in the vertebral column. The space between adjacent vertebral bodies required for housing the implant is adapted approximately to the shape of the implant in order to achieve a positive fit between the adjacent vertebrae, which after the operation produces a certain primary stability until finally the adjacent osseous tissue grows into the open-pore surface structure and reinforces the adjacent vertebral bodies with respect to one another in a rotationally secure manner. A disadvantage of this is the fact that a relatively large bone volume is resected when adjacent vertebral members still show no or only slight evidence of wear.

The object of the invention is therefore to provide an implant of the aforementioned type which enables an effective reinforcement of adjacent vertebral bodies with an implantation procedure which conserves bone.

This object is achieved in accordance with the invention with the implant of the aforementioned type in that the implant contains several spherical spacers made from open-cell or open-pore metal, which are disposed on at least one connecting element.

The advantages of the invention lie in particular in that the spherical spacers, which are connected to one another by means of the connecting element, have such a small radius that the implant can be inserted between the facing bearing surfaces of two adjacent vertebrae so that the spherical spacers abut the two bearing surfaces and the original distance between the bearing surfaces can be reproduced without a noticeable or larger quantity of bone material having to be resected. The spherical spacers come to lie between the natural bearing surfaces of the adjacent vertebrae and after the operation guarantee a mobility of the vertebral bodies relative to one another which decreases as the osseous tissue increasingly grows into the open-pore metal structure and finally stops when the osseous tissue has securely grown into the spacers. The fact that the only preparation that the facing bearing surfaces of the vertebral bodies have to undergo is gentle to the bones and does not require any greater bone resection is a particular advantage.

According to a particularly preferred embodiment of the invention, the connecting element, on which the spherical spacers are disposed, is made from a bioabsorbable material, which is gradually broken down in the body, so that in the implanted state the connecting element disintegrates, and only the spherical spacers between the adjacent bearing surfaces remain.

According to a further preferred embodiment of the invention, the spacers contain a central bore. The connecting element is made from a bioabsorbable thread which extends through the bores of the spacers. The spacers are preferably attached to the bioabsorbable thread at a distance from one another, and the thread can have a predetermined inherent stability, for example a circular shape, which gives the spherical spacers fixed positions with respect to one another.

In a particularly preferred manner all the spherical spacers possess the same diameter and are flattened at two opposing poles, which come to abut the bearing surfaces of the vertebral bodies. By this flattening at the poles, the relative primary mobility of the adjacent vertebral bodies is restricted, as the spacers then no longer perform any rotational movements - under the bearing pressure acting in the direction of the vertebral column.

At least three or four spherical spacers are advantageously attached to the connecting element in order to produce a three-point or four-point bearing between the adjacent vertebral bodies.

Refinements of the invention are characterised by the features of the subordinate claims.

An exemplified embodiment of the invention is explained in further detail below by means of the drawings.
- Figure 1: shows a lateral elevation of the implant between two adjacent vertebral bodies;
- Figure 2: shows a plan view of the implant according to Figure 1;
- Figure 3: shows a lateral elevation of a spherical spacer with flattened poles.

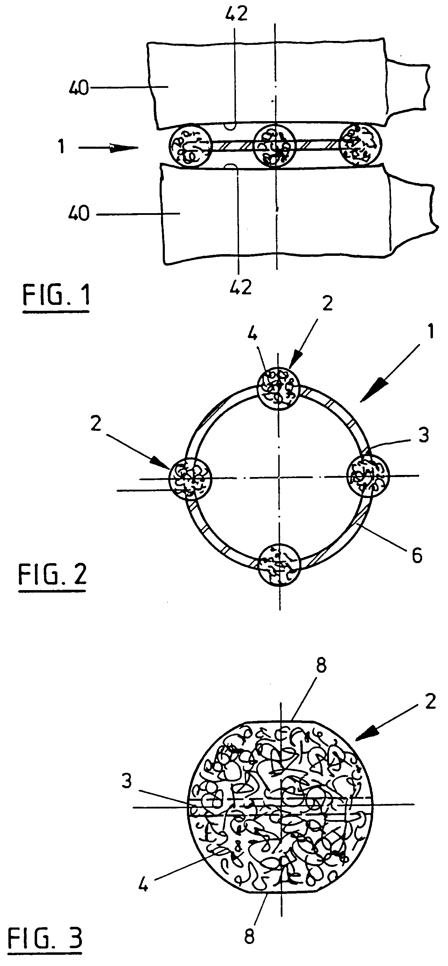

Figures 1 and 2 show an implant 1 for fixing adjacent vertebrae in lateral elevation (Figure 1) and in plan view (Figure 2). Several spherical spacers 2 in the embodiment 4 represented having an open-cell or open-pore metal structure are attached to a connecting element 6, for example a thread made from bioabsorbable material. The spacers 2 have central bores 3, through which the connecting element 6 is passed. The spacers 2 are attached to the thread 6 at a distance from one another and all have roughly the same diameter.

In the embodiment shown in Figure 3 the spherical spacers 2 are flattened at two poles 8 which are opposing one another. The flattened spacers 2 are attached to the connecting element 6 in such a manner that the flattened poles 8 lie in one plane above and below the connecting element and in the implanted state - shown in Figure 1 - abut against the bearing surface 42 of adjacent vertebral bodies 40.

In the embodiment shown the spacers are throughout constructed as members having an open-cell or open-pore metal structure. Alternatively the spacers may also be made with a core of solid metal, which on its surface bears the open-pore or open-cell structure.

## Claims

1. An implant for fixing adjacent vertebrae **characterised in that** the implant comprises several spherical spacers (2) having an open-cell or open-pore metal structure, which are disposed on at least one connecting element (6).

2. An implant according to Claim 1, **characterised in that** the connecting element (6) is made from bioabsorbable material.

3. An implant according to Claim 1 or 2, **characterised in that** the spacers (2) contain a central bore (3), **and in that** the connecting element (6) is a bioabsorbable thread which extends through the bores (3) of the spacers (2).

4. An implant according to Claim 3, **characterised in that** the spacers (2) are attached to the thread (6) at a distance from one another.

5. An implant according to one of the preceding Claims, **characterised in that** the two ends of the thread (6) are connected to one another.

6. An implant according to one of the preceding Claims, **characterised in that** the spherical spacers (2) all have the same diameter.

7. An implant according to one of the preceding Claims, **characterised in that** the spherical spacers (2) are flattened on two opposing poles (8).
